# EUROPEAN PATENT SPECIFICATION

(11) **EP 4 031 054 B1**
(45) Date of publication and mention of the grant of the patent: **09.09.2026**
(21) Application number: 20868027.2
(22) Date of filing: 28.09.2020
(51) Int. Cl.: A61B 50/30

(54) **INTRAOSSEOUS STERILE BARRIER AND PACKAGING**
INTRAOSSÄRE STERILE BARRIERE UND VERPACKUNG
BARRIÈRE STÉRILE INTRAOSSEUSE ET EMBALLAGE

(30) Priority: 27.09.2019 US 201962907446 P
(43) Date of publication of application: 27.07.2022
(73) Proprietor: Bard Access Systems, Inc., Salt Lake City, UT 84116 (US)
(72) Inventor: LINDEKUGEL, Eric, W., Salt Lake City, UT 84106 (US); BLANCHARD, Daniel, B., Bountiful, UT 84010 (US)
(74) Representative: Hoffmann Eitle
(86) International application number: PCT/US2020/053081
(87) International publication number: WO 2021/062368

(56) References cited:
- CA-A1- 3 044 117
- US-A- 5 868 711
- US-A1- 2005 165 404
- US-A1- 2006 015 066
- US-A1- 2008 140 014
- US-A1- 2010 312 246
- US-A1- 2010 312 246
- US-A1- 2011 082 387
- US-A1- 2015 129 456
- US-A1- 2018 125 465
- US-B1- 6 716 215

## Description

### SUMMARY

Claims 1 and 8 define the invention and dependent claims disclose embodiments. Briefly summarized, embodiments disclosed herein are directed to apparatus and methods for a sterile barrier and packaging assembly for an intraosseous access device. Currently, intraosseous needles, and associated access assemblies, are provided as individually packaged items that require both a protective covering and a separate, sterile barrier disposed thereover. This requires a user to remove the access assembly from one or more outer packagings, affix the assembly to the driving mechanism, then remove the cover prior to insertion. These extra steps of removing additional packaging costs valuable time when deploying intraosseous devices in emergency situations.
CA 3 044 117 A1 relates to a storage device for storing a pen needle assembly including a housing having first wells with a dimension for receiving the pen needle assembly and second wells for receiving an inner shield of the pen needle assembly.
US 2006/015066 A1 relates to a device for infusion or aspiration that includes a base and at least one needle positioned within the base, where the base includes one or more locators for positioning the infusion device in relation to one or more predetermined anatomical features. US 2010/312246 A1 relates to some introducer tools for bone portals having a handle coupled to a driving member by a force-limiting coupling. US2015/129456 A1, US2005/165404 A1, US5868711 A and US2003/029763 A1 disclose devices of the prior art.

Disclosed herein, in some embodiments, are apparatuses and methods that incorporate both mechanical protection and a sterile barrier in a single packaging item that is quick and easy to deploy. In some embodiments, the driving mechanism and access assembly are incorporated into one disposable device. Further, the access device can be retractable into the disposable driver, reducing the overall size of the device. This provides a highly portable system that is readily available for users in emergency situations. Further, the steps and time required to unpackage and assemble the device are reduced.

Disclosed herein in some embodiments is an intraosseous system including a sterile packaging for containing the access assembly of the intraosseous system, the sterile packaging including a cavity configured to receive the access assembly, a closed distal end, a proximal opening in communication with the cavity, and a lid disposed over the proximal opening to seal the access assembly in the cavity.

In some embodiments, the lid provides a sterile barrier to maintain the access assembly in a sterile environment. The proximal opening is configured to receive a distal portion of the driver therethrough to engage the access assembly. The cavity defines a first cross-sectional area configured to receive a needle portion of the access assembly, and a second cross-sectional area larger than the first cross-sectional area configured to receive a hub portion of the access assembly. The second cross-sectional area releasably engages the hub portion of the access assembly in an interference engagement, protrusion and detent engagement, threaded engagement, or adhesive engagement. The second cross-sectional area includes an O-ring configured to engage the hub portion of the access assembly provide a seal between a distal portion the cavity and a proximal portion of the cavity.

In some embodiments, the lid is formed of a gas-permeable, organic material, synthetic material, woven material, non-woven material, polymer, co-polymer, olefin fiber, polyethylene, high density polyethylene ("HOPE"), or TYVEK^{®}. The lid provides a physical and sterile barrier as well as allowing for EtO sterilization of the access assembly disposed within the cavity. The lid is releasably coupled to the proximal opening with one of heat-sealing, bonding, adhesive, or welding. The lid includes one of a finger loop or a pull tab configured to facilitate separation of the lid from the sterile packaging. The sterile packaging includes a flange extending radially from a proximal edge of the cap. The lid includes a tear line configured to allow a first portion of the lid to separate from a second portion of the lid and allow the access assembly to pass through the proximal opening. The sterile packaging further includes a fin extending longitudinally along an outer surface thereof and configured to provide structural support.

Also disclosed as an aspect not part of the presently claimed invention is an intraosseous access device including, a driver, an access assembly coupled to the driver, and a sterile packaging defining a cavity, coupled to a portion of the driver, and enclosing the access assembly to maintain the access assembly in a sterile environment.

In some embodiments, a proximal end of the sterile packaging is releasably secured to the driver by heat-sealing, bonding, adhesive, or welding. A proximal end of the sterile packaging is releasably secured to the driver by a threaded engagement between an inner surface of the sterile packaging and an outer surface of the driver. A proximal end of the sterile packaging includes a tear-away strip configured detach from one of the sterile packaging or the driver and release the sterile packaging from the driver. One of the sterile packaging or the driver includes an opening communicating with the cavity of the sterile packaging and including a lid disposed thereover to maintain the sterile environment, the lid formed of a different material from the sterile packaging or the driver.

In some embodiments, the lid is formed of a gas-permeable, organic material, synthetic material, woven material, non-woven material, polymer, co-polymer, olefin fiber, polyethylene, high density polyethylene ("HDPE"), or TYVEK^{®}. The lid provides a physical and sterile barrier as well as allowing for EtO sterilization of the access assembly disposed therein. The lid is coupled to the sterile packaging with one of heat-sealing, bonding, adhesive, or welding. The sterile packaging or the driver includes a fin extending longitudinally along an outer surface thereof and configured to provide a structural support or to provide a gripping feature for rotation of the sterile packaging relative to the driver. The access assembly is transitionable between a retracted state and a deployed state, where a portion of the access assembly is disposed within the driver in the retracted state.

In some embodiments, the sterile packaging is coupled to the access assembly and configured to transition the access assembly from the retracted state to the deployed state when the sterile packaging is removed. In some embodiments, the intraosseous access device further includes an extension set releasably retained within the cavity defined the sterile packaging to maintain the extension set within a sterile environment. In some embodiments, the intraosseous access device further includes an extension set releasably retained within a cavity defined by the body of the driver, and including a lid disposed over an opening to the cavity to provide a sterile barrier and maintain the extension set within a sterile environment.

Also disclosed is a method of preparing an intraosseous access device for use including, providing an access assembly disposed within a cavity of a sterile packaging, and including a lid disposed over a proximal opening that communicates with the cavity, to provide a sterile environment therein, urging a coupling interface of an intraosseous driver through the proximal opening to engage a coupling interface of the access assembly, and withdrawing the sterile packaging distally to expose the access assembly.

In some embodiments, the method further includes disengaging a hub portion of the access assembly from an interior surface of the sterile packaging, the hub portion engaging the sterile packaging in an interference engagement, protrusion and detent engagement, threaded engagement, or adhesive engagement. In some embodiments, the cavity includes an O-ring disposed on an inner surface thereof and configured to engage the hub portion to provide a seal between a distal portion of the sterile packaging and a proximal portion of the sterile packaging. The lid is formed of a gas-permeable material, organic material, synthetic material, woven material, non-woven material, polymer, co-polymer, olefin fiber, polyethylene, high density polyethylene ("HDPE"), or TYVEK^{®}.

In some embodiments, the lid provides a physical and sterile barrier as well as allowing for EtO sterilization of the sterile packaging, and the access assembly disposed therein. The lid is coupled to the sterile packaging with one of heat-sealing, bonding, adhesive, or welding. In some embodiments, the method further includes removing the lid from the sterile packaging prior to urging the coupling interface of the driver through the proximal opening. In some embodiments, the method further includes pulling one of a finger loop or a pull tab coupled to the lid to separate the lid from the sterile packaging. In some embodiments, the method further includes urging the coupling interface of the driver through a tear line disposed in the lid, the tear line configured to separate a first portion of the lid from a second portion of the lid to allow the coupling interface of the driver to pass therebetween. The sterile packaging includes a flange extending radially from a proximal edge of the sterile packaging.

Also disclosed as an aspect not part of the presently claimed invention is a method of accessing a medullary cavity including, providing an intraosseous driver including an access assembly extending from a distal portion thereof, and a sterile packaging coupled to the distal portion and defining a cavity, the sterile packaging configured to enclose the access assembly within a sterile environment, removing the sterile packaging, and accessing the medullary cavity.

In some embodiments, the sterile packaging is releasably secured to the distal portion of the intraosseous driver by heat-sealing, bonding, adhesive, or welding. The sterile packaging is releasably secured to the intraosseous driver by a threaded engagement between an inner surface of the sterile packaging and an outer surface of the intraosseous driver. In some embodiments, the method further includes detaching a tear-away strip configured detach from one of the sterile packaging or the intraosseous driver and release the sterile packaging from the intraosseous driver. In some embodiments, one of the sterile packaging or the intraosseous driver includes an opening communicating with the cavity and further including a lid disposed over the opening to maintain the sterile environment, the lid formed of a different material from one of the sterile packaging or the intraosseous driver.

The lid is formed of a gas-permeable, organic material, synthetic material, woven material, non-woven material, polymer, co-polymer, olefin fiber, polyethylene, high density polyethylene ("HDPE"), or TYVEK^{®}. The lid provides a physical and sterile barrier as well as allowing for EtO sterilization of the access assembly disposed therein. The lid is coupled to the sterile packaging or the intraosseous driver with one of heat-sealing, bonding, adhesive, or welding. In some embodiments, the method further includes transitioning the access assembly from a retracted state to a deployed state, where a portion of the access assembly is disposed within a handle of the intraosseous driver in the retracted state. In some embodiments, removing the sterile packaging transitions the access assembly from the retracted state to the deployed state. In some embodiments, the method further includes removing an extension set releasably retained within the cavity defined by the sterile packaging and coupling the extension set to a needle hub of the access assembly. In some embodiments, the method further includes removing a second lid disposed over a second opening communicating with a second cavity defined by the intraosseous driver, and removing an extension set releasably retained therein and coupling the extension set to a needle hub of the access assembly.

Also disclosed is a method of sterilizing an intraosseous access system including, coupling an access assembly to a driver, enclosing the access assembly within an interior cavity of a sterile packaging, releasably coupling the sterile packaging with the driver, and sterilizing the intraosseous access system.

In some embodiments, one of the sterile packaging or the driver includes an opening communicating with the interior cavity of the sterile packaging and including a lid disposed thereover to maintain a sterile barrier and configured to allow EtO sterilization of the interior cavity. In some embodiments, the method further includes an extension set disposed within the interior cavity of the sterile packaging or an interior cavity of the driver.

These and other features of the concepts provided herein will become more apparent to those of skill in the art in view of the accompanying drawings and following description, which disclose particular embodiments of such concepts in greater detail.

### DRAWINGS

FIG. 1 illustrates an exploded view of an embodiment of an intraosseous access system, wherein an access assembly subset of the system is depicted slightly enlarged and in elevation, and an automated driver component is depicted in perspective, in accordance with embodiments disclosed herein;
FIGS. 2A-2B illustrate perspective views of a sterile barrier and packaging assembly, in accordance with embodiments disclosed herein;
FIG. 3 illustrates an intraosseous access device including a sterile barrier and packaging assembly, in accordance with embodiments disclosed herein.
FIG. 4 illustrates an intraosseous access device including a sterile barrier and packaging assembly, in accordance with embodiments disclosed herein.
FIG. 5 illustrates an intraosseous access device including a sterile barrier and packaging assembly, in accordance with embodiments disclosed herein.

### DESCRIPTION

Before some particular embodiments are disclosed in greater detail, it should be understood that the particular embodiments disclosed herein do not limit the scope of the concepts provided herein. It should also be understood that a particular embodiment disclosed herein can have features that can be readily separated from the particular embodiment and optionally combined with or substituted for features of any of a number of other embodiments disclosed herein.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

With respect to "proximal," a "proximal portion" or a "proximal end portion" of, for example, a needle disclosed herein includes a portion of the needle intended to be near a clinician when the needle is used on a patient. Likewise, a "proximal length" of, for example, the needle includes a length of the needle intended to be near the clinician when the needle is used on the patient. A "proximal end" of, for example, the needle includes an end of the needle intended to be near the clinician when the needle is used on the patient. The proximal portion, the proximal end portion, or the proximal length of the needle can include the proximal end of the needle; however, the proximal portion, the proximal end portion, or the proximal length of the needle need not include the proximal end of the needle. That is, unless context suggests otherwise, the proximal portion, the proximal end portion, or the proximal length of the needle is not a terminal portion or terminal length of the needle.

With respect to "distal," a "distal portion" or a "distal end portion" of, for example, a needle disclosed herein includes a portion of the needle intended to be near or in a patient when the needle is used on the patient. Likewise, a "distal length" of, for example, the needle includes a length of the needle intended to be near or in the patient when the needle is used on the patient. A "distal end" of, for example, the needle includes an end of the needle intended to be near or in the patient when the needle is used on the patient. The distal portion, the distal end portion, or the distal length of the needle can include the distal end of the needle; however, the distal portion, the distal end portion, or the distal length of the needle need not include the distal end of the needle. That is, unless context suggests otherwise, the distal portion, the distal end portion, or the distal length of the needle is not a terminal portion or terminal length of the needle.

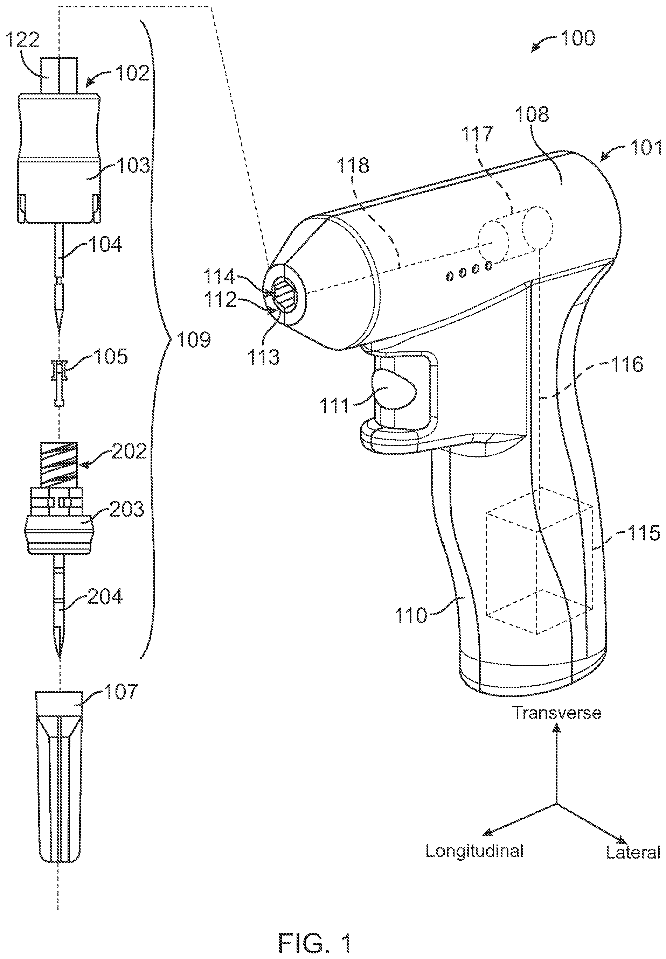

As shown in FIG. 1, and to assist in the description of embodiments described herein, a longitudinal axis extends substantially parallel to an axial length of a needle extending from the driver. A lateral axis extends normal to the longitudinal axis, and a transverse axis extends normal to both the longitudinal and lateral axes.

Unless defined otherwise, all technical and scientific terms used herein have the same meaning as commonly understood by those of ordinary skill in the art.

The present disclosure generally relates to intraosseous (IO) access devices, systems, and methods thereof. In particular, embodiments disclosed herein include sterile barriers and packaging for components of IO devices.

FIG. 1 shows an exploded view of an exemplary embodiment of an intraosseous access system 100, with some components thereof shown in elevation and some components shown in perspective. The intraosseous access system 100 can be used to penetrate skin and underlying hard bone for intraosseous access, such as, for example to access the marrow of the bone and/or a vasculature of the patient via a pathway through an interior of the bone.

In an embodiment, the system includes a driver 101 and an access assembly 109. The driver 101 can be used to rotate the access assembly 109 into a bone of a patient. In embodiments, the driver 101 can be automated or manual. In an embodiment, the driver 101 is an automated driver 108. For example, the automated driver 108 can be a drill that achieves high rotational speeds.

The intraosseous access system 100 can further include an obturator assembly 102, a shield 105, and a needle assembly 202, which may be referred to, collectively, as the access assembly 109. The access assembly 109 may also be referred to as an access system. The obturator assembly 102 is referred to as such herein for convenience. In an embodiment, the obturator assembly 102 includes an obturator 104. However, in some embodiments, the obturator 104 may be replaced with a different elongated medical instrument. As used herein, the term "elongated medical instrument" is a broad term used in its ordinary sense that includes, for example, such devices as needles, cannulas, trocars, obturators, stylets, and the like. Accordingly, the obturator assembly 102 may be referred to more generally as an elongated medical instrument assembly. In like manner, the obturator 104 may be referred to more generally as an elongated medical instrument.

In an embodiment, the obturator assembly 102 includes a coupling hub 103 that is attached to the obturator 104 in any suitable manner (e.g., one or more adhesives or overmolding). The coupling hub 103 can be configured to interface with the driver 101, as further discussed below. The coupling hub 103 may alternatively be referred to as an obturator hub 103 or, more generally, as an elongated instrument hub 103.

In an embodiment, the shield 105 is configured to couple with the obturator 104. The coupling can permit relative longitudinal movement between the obturator 104 and the shield 105, such as sliding, translating, or other movement along an axis of elongation (i.e., axial movement), when the shield 105 is in a first operational mode, and can prevent the same variety of movement when the shield 105 is transitioned to a second operational mode. For example, the shield 105 may couple with the obturator 104 in a manner that permits longitudinal translation when the obturator 104 maintains the shield 105 in an unlocked state, and when the obturator 104 is moved to a position where it no longer maintains the shield in the unlocked state, the shield 105 may automatically transition to a locked state in which little or no translational movement is permitted between the shield 105 and the obturator 104. Stated otherwise, the shield 105 may be longitudinally locked to a fixed or substantially fixed longitudinal orientation relative to the obturator 104 at which the shield 105 inhibits or prevents inadvertent contact with a distal tip of the obturator, as further discussed below. In various embodiments, the shield 105 may be configured to rotate relative to the obturator 104 about a longitudinal axis of the obturator 104 in one or more of the unlocked or locked states.

With continued reference to FIG. 1, the needle assembly 202 is referred to as such herein for convenience. In an embodiment, the needle assembly 202 includes a needle 204. However, in various other embodiments, the needle 204 may be replaced with a different instrument, such as, for example, a cannula, a tube, or a sheath, and/or may be referred to by a different name, such as one or more of the foregoing examples. Accordingly, the needle assembly 202 may be referred to more generally as a cannula assembly or as a tube assembly. In like manner, the needle 204 may be referred to more generally as a cannula.

In an embodiment, the needle assembly 202 includes a needle hub 203 that is attached to the needle 204 in any suitable manner. The needle hub 203 can be configured to couple with the obturator hub 103 and may thereby be coupled with the driver 101, as further discussed below. The needle hub 203 may alternatively be referred to as a cannula hub 203.

In an embodiment, the shield 105 is configured to couple with the needle hub 203. The coupling can prevent relative axial or longitudinal movement between the needle hub 203 and the shield 105, such as sliding, translating, or the like, when the shield 105 is in the first operational mode, and can permit the shield 105 to decouple from the needle hub 203 when the shield 105 is transitioned to the second operational mode. For example, as further discussed below, the shield 105 may couple with the needle hub 203 so as to be maintained at a substantially fixed longitudinal position relative thereto when the obturator 104 maintains the shield 105 in the unlocked state, and when the obturator 104 is moved to a position where it no longer maintains the shield in the unlocked state, the shield 105 may automatically transition to a locked state relative to the obturator 104, in which state the shield 105 also decouples from the needle hub 203.

In an embodiment, the shield 105 can be coupled with the obturator 104, the obturator 104 can be inserted into the needle 204, and the obturator hub 103 can be coupled to the needle hub 203 to assemble the access assembly 109. In an embodiment, a cap 107 may be provided to cover at least a distal portion of the needle 204 and the obturator 104 prior to use of the access assembly 109. For example, in an embodiment, a proximal end of the cap 107 can be coupled to the obturator hub 103.

With continued reference to FIG. 1, the automated driver 108 may take any suitable form. The driver 108 may include a handle 110 that may be gripped by a single hand of a user. The driver 108 may further include an actuator 111 of any suitable variety via which a user may selectively actuate the driver 108 to effect rotation of a coupling interface 112. For example, the actuator 111 may comprise a button, as shown, or a switch or other mechanical or electrical element for actuating the driver 108. In an embodiment, the coupling interface 112 is formed as a socket 113 that defines a cavity 114. The coupling interface 112 can be configured to couple with the obturator hub 103. In an embodiment, the socket 113 includes sidewalls that substantially define a hexagonal cavity into which a hexagonal protrusion of the obturator hub 103 can be received. Other suitable connection interfaces are contemplated.

The automated driver 108 can include an energy source 115 of any suitable variety that is configured to energize the rotational movement of the coupling interface 112. For example, in some embodiments, the energy source 115 may comprise one or more batteries that provide electrical power for the automated driver 108. In other embodiments, the energy source 115 can comprise one or more springs (e.g., a coiled spring) or other biasing member that may store potential mechanical energy that may be released upon actuation of the actuator 111.

The energy source 115 may be coupled with the coupling interface 112 in any suitable manner. For example, in an embodiment, the automated driver 108 includes an electrical, mechanical, or electromechanical coupling 116 to a gear assembly 117. In some embodiments, the coupling 116 may include an electrical motor that generates mechanical movement from electrical energy provided by an electrical energy source 115. In other embodiments, the coupling 116 may include a mechanical linkage that mechanically transfers rotational energy from a mechanical (e.g., spring-based) energy source 115 to the gear assembly 117. The automated driver 108 can include a mechanical coupling 118 of any suitable variety to couple the gear assembly 117 with the coupling interface 112. In other embodiments, the gear assembly 117 may be omitted.

In embodiments, the automated driver 108 can rotate the coupling interface 112, and thereby, can rotate the access assembly 109 at rotational speeds significantly greater than can be achieved by manual rotation of the access assembly 109. For example, in various embodiments, the automated driver 108 can rotate the access assembly 109 at speeds of between 200 and 3,000 rotations per minute (rpm). However, greater or lesser rotational speeds are also contemplated.

Further details and embodiments of intraosseous access systems can be found in WO 2018/075694, WO 2018/165334, WO 2018/165339, and US 2018/0116693.

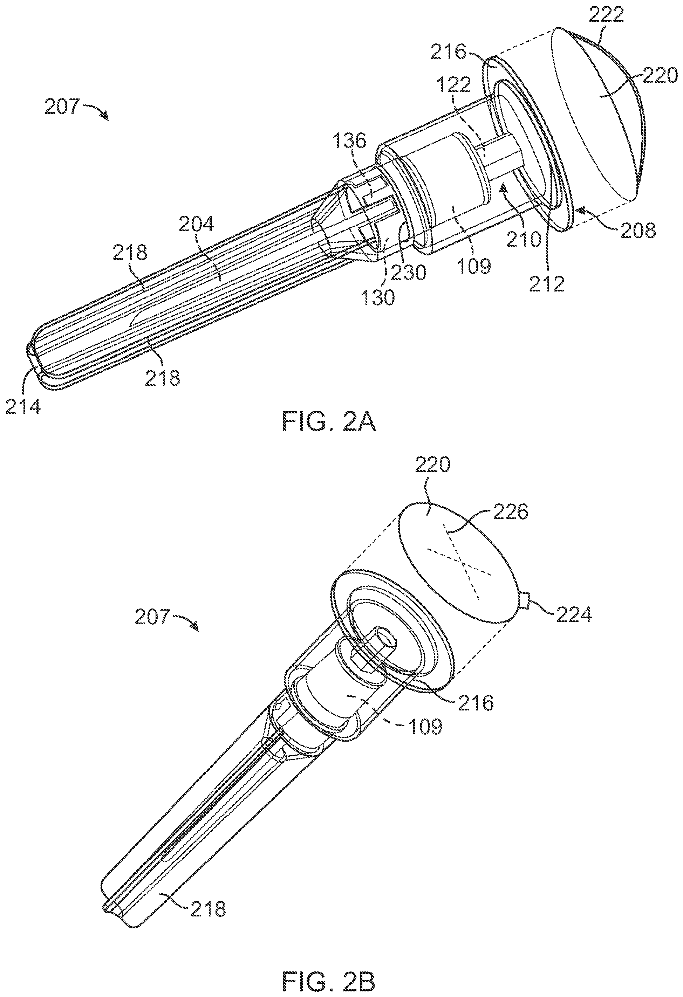

FIGS. 2A-2B show an embodiment of a sterile packaging, e.g. cap 207, with an exemplary access assembly 109 disposed therein. The cap 207 defines a substantially cylindrical cavity 210 extending along a longitudinal axis, from a distal end 214 to a proximal end 212, and communicating with an opening 208 disposed at the proximal end 212. To note, the distal end 214 of the cap 207 is sealed and is formed as a single unitary piece with the cap 207. As shown, the cavity 210 defines a substantially circular cross-sectional shape, although it will be appreciated that other cross-sectional shapes are also contemplated and fall within the scope of the present invention. In an embodiment, the cylindrical cavity 210 defines a substantially uniform cross-sectional area along the longitudinal length thereof. In an embodiment, the cylindrical cavity 210 includes portions defining different cross-sectional areas along the longitudinal length thereof.

In an embodiment, the proximal opening 208 and a proximal portion of the cylindrical cavity 210 includes a cross-sectional area sufficient to receive a coupling interface 112 of the driver 101, such that a distal portion of the driver 101 can be inserted through the opening 208 and a driver coupling interface 112 can engage a coupling interface 122 of the access assembly 109.

In an embodiment, the exemplary access assembly 109 is releasably secured within the cap 207 by way of mechanical interference engagement, protrusion and detent engagement, threaded engagement, adhesive, combinations thereof, and the like. For example, in an embodiment, the obturator hub 103 further includes a pair of outward protrusions 136 that can engage an inner surface of the cap 207 and can assist in coupling the access assembly 109 to the cap 207. In an embodiment, the cap 207, or a portion thereof, can define an inner diameter only slightly larger than an outer diameter of the skirt 130 of the hub 103. The outward protrusions 136 can slightly deform a proximal end of the cap 207 from a substantially cylindrical shape to a more oblong shape, which may enhance a grip of the cap 207 against the skirt 130. Any other suitable connection arrangement for the cap 207 is also contemplated.

In an embodiment, the cap 207 can include a rubber O-ring 230, grommet, or similar structure disposed within a wall of the cap 207 and configured to engage a hub of the access assembly 109, e.g. the obturator hub 103, when the access assembly 109 is disposed within the cap 207. In an embodiment, the O-ring 230 can be configured to retain the access assembly 109 within the cap 207. In an embodiment, the O-ring 230 can provide a seal between cap 207 and the access assembly 109 to maintain the needle 204, disposed distally of the O-ring 230, in a sterile environment even if the proximal opening 208 is opened, as discussed in more detail herein. Advantageously, the seal can maintain the needle 204 in a sterile environment, even with the driver 101 is coupled to the access assembly 109, and maintain the needle 204 in a sterile environment right up until the moment of use, mitigating the introduction of pathogens or the like.

In an embodiment, the proximal end 212 of the cap 207 includes a flange 216 extending radially about the longitudinal axis, from a proximal edge of the cap 207. In an embodiment, the flange 216 provides a distally facing surface that allows a user to grasp the cap 207 and urge the access assembly 109 disposed therein, proximally onto a coupling interface 112 of a driver 101. This can be of particular importance when a user is wearing gloves or has limited grip or dexterity. In an embodiment, the flange 216 provides a proximally facing surface for a lid 220 to be coupled thereto, as discussed in more detail herein. In an embodiment, the cap 207 includes one or more fins 218, disposed on an outer surface of the cap 207, extending parallel to a longitudinal axis. The fins 218 provide mechanical strength to the cap 207, preventing deformation of the cap 207 and protecting the access assembly 109, disposed therein from damage. Further, the fins 218 provide a gripping feature allowing the user to grasp the cap 207 securely. It will be appreciated that the outer surface of the cap 207 can further include additional textured surfaces, additional materials including high frictional co-efficient, combinations thereof, or the like, to improve user grip with the outer surface of the cap 207.

In an embodiment, the cap 207 is formed of a substantially resilient material such as a metal, alloy, plastic, polymer, co-polymer, thermoplastic, polypropylene, composite, carbon composite, combinations thereof, or the like. In an embodiment, the material that forms the cap 207 can be substantially rigid and resistant to deformation. In an embodiment, the material that forms the cap 207 can form a sterile barrier, can be sterilized, or is gas impermeable. Sterilization techniques contemplated herein include Ethylene Oxide ("EtO") sterilization, Gamma irradiation ("Gamma") sterilization, electron beam irradiation ("E-Beam") sterilization, or similar sterilization techniques known in the art. In an embodiment, the cap 207 also includes a lid 220 attached to a proximal end 212 of the cap 207. The lid 220 can be coupled to the cap 207 using heat-sealing, bonding, adhesive, welding, or similar suitable techniques that releasably secures the lid 220 to the cap 207. In an embodiment, the lid 220 is attached to a proximally-facing surface of the flange 216.

In an embodiment, the lid 220 can be formed of a gas-permeable or gas-impermeable material, organic or synthetic, woven or non-woven material, including polymers, co-polymers, olefin fibers, polyethylene, high density polyethylene ("HDPE"), TYVEK^{®}, combinations thereof, or the like. The lid 220 can provide a physical and sterile barrier. In an embodiment, the lid 220 can be gas permeable to allow for Ethylene Oxide ("EtO") sterilization of the cap 207, and any contents disposed therein, when the lid 220 is disposed over the proximal opening 208 of the cap. In an embodiment, the lid 220 can provide a gas impermeable barrier to provide a physical and sterile barrier, for example, where Gamma or E-Beam sterilization techniques are be used.

In an embodiment, the lid 220 includes a gripping feature that allows the user to grasp a portion of the lid 220 and peel the lid 220 away from the cap 207 to access the contents therein. In an embodiment, the gripping feature includes a tab, finger loop, or similar structure, or combinations thereof. In an embodiment, the gripping feature includes a finger loop 222 extending from a proximal surface of the lid 220. In an embodiment, the gripping feature includes a tab 224 extending from a perimeter of the lid 220. Although it will be appreciated that similar structures, or combinations thereof, are contemplated to fall within the scope of the present invention.

In an embodiment, the lid 220 includes a tear line 226. The tear line 226 can include score-lines, perforations, laser cut lines, or similar lines of weakness that allow for separation of a portion of the lid 220 there along. In an embodiment, a user can urge an item, e.g. a distal portion of the driver 101, through the tear line 226 of the lid 220 to access the access assembly 109 disposed therein. Advantageously, this expedites attaching the access assembly 109 to the driver 101 by obviating the need to remove the lid 220 first. Instead, a user urges the driver 101 through the lid 220, breaking tear line 226, until driver coupling interface 112 engages the access assembly coupling interface 122. The cap 207 can then be withdrawn distally exposing the needle 204 ready of use.

In an embodiment, the tear line 226 can facilitate the removal of the lid 220 prior to affixing the access assembly 109 to a driver 101. For example, a user can pull on a gripping feature, e.g. finger loop 222 or tab 224, which can cause a portion of the lid 220 to detach along a tear line 226. With a portion of the lid 220 removed, a proximal portion of the access assembly 109 is exposed to allow a user to couple the driver therewith, e.g. by coupling the driver interface 112 with the access assembly coupling interface 122. To note, the intact lid 220, including the tear line 226, can still maintain a sterile barrier between the cavity 210 and the surrounding environment.

Advantageously, the cap 207 allows the access assembly 109 to be individually wrapped and serves both as protection during shipping and handling of the access assembly 109, as well as a sterile barrier, and does not require the user to remove the device from a separate sterile barrier packaging. While embodiments of the cap 207 are shown as containing an exemplary access assembly 109, it will be appreciated this is not limited only to the access assembly 109, and in some embodiments, the cap 207 can also contain individual components of the access assembly 109, or combinations thereof, or can contain additional components of the intraosseous access system 100. Further the cap 207 allows the driver 101, or similar placement device, to access the access assembly 109 without the need to physically touch the access assembly 109, thus eliminating the risk of contamination and reducing the number of packaging components, e.g. additional Tyvek pouches and the like. Advantageously, embodiments disclosed herein expedites attachment of the access assembly 109 to the driver 101 while maintaining a sterile barrier up until the moment of use.

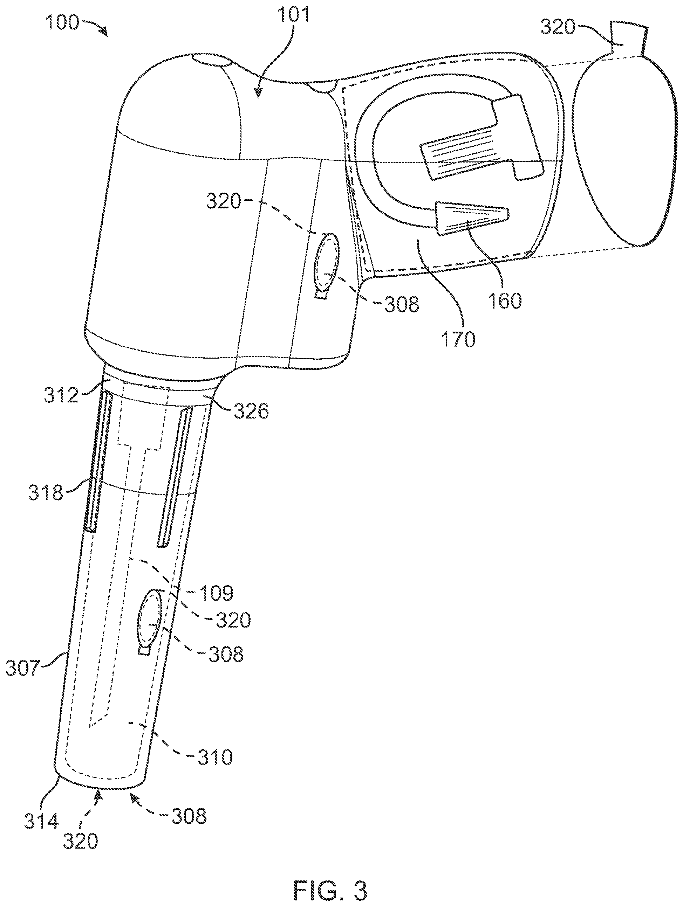

As shown in FIG. 3, in an embodiment, the intraosseous access system 100 includes a sterile packaging, such as cap 307, provided as a single-use system, with the access assembly 109 already coupled with the driver 101. The cap 307 serves both as protection during shipping and handling, as well as a sterile barrier between the surrounding environment and portions of the system 100 that make contact with the patient.

In an embodiment a cap 307 is provided and defines a substantially cylindrical cavity 310, extending along a longitudinal axis, from a distal end 314 to a proximal end 312. The cap 307 is configured to contain an access assembly 109, and includes similar features to embodiments described herein. It will be appreciated that the access assembly 109 is exemplary and one of ordinary skill in the art will appreciate that the cap 307 protects at least a portion of the intraosseous access system 100 that contacts a patient during use.

In an embodiment, a proximal end 312 of the cap 307 is configured to engage a distal portion of the driver 101, including the driver coupling interface 112. The proximal end 312 can be releasably secured to the driver 101, for example by heat-sealing, bonding, adhesive, welding, or similar suitable techniques. In an embodiment, the cap 307 is coupled to the driver 101 by way of a threaded engagement between an inner surface of the cap 307 and an outer surface of the driver 101. In use, the user grasps outer surface of the cap 307 and can rotate the cap 307 relative to the driver 101 about the longitudinal axis, twisting to break the releasable securement, threaded portion, or combinations thereof, to release the cap 307 from the driver 101. In an embodiment, the cap 307 include fins 318 that provide mechanical strength to the cap 307 and provide a gripping feature. It will be appreciated that the outer surface of the cap 307 can further include additional textured surfaces, additional materials including high frictional co-efficient, combinations thereof, and the like to improve user grip with the outer surface of the cap 307.

In an embodiment, the cap 307 includes a tear away strip 326 that is defined by one or more tear lines, and includes a gripping feature, such as a tab or finger loop, as described herein. In use, a user pulls the gripping feature which causes the tear away strip 326 to separate from the intraosseous access system 100 and causes the cap 307 to separate from the driver 101. In an embodiment, a distal end 314 of the cap 307 is formed as a continuous sealed end to define a cavity 310 therein. In an embodiment, the cavity 310, and access assembly 109 disposed therein, is sterilized prior to assembly the driver 101.

In an embodiment, the cap 307 includes an opening 308 that communicates with an interior cavity 310 of the cap 307 and can include a lid 320 disposed thereover. In an embodiment, the opening 308 is disposed at a distal end 314 of the cap 307 although it will be appreciated that the opening 308 can be disposed through a sidewall of the cap 307. In an embodiment, the opening 308 can be disposed in a wall of a body of the driver 101 to communicate with an interior cavity of the body. To note, the interior cavity of the body can be in communication with an interior cavity 310 of the cap 307. It will be appreciated that other combinations of one or more openings 308 communicating with an interior portion of the driver 101 or cap 307 or also contemplated.

In an embodiment, the lid 320 can be formed of a gas permeable material that allows EtO sterilization of the cavity 310 and any components disposed therein after the cap 307, access assembly 109, and driver 101 have been assembled. In an embodiment, the lid 320 may be gas impermeable, for example where Gamma or E-Beam sterilization techniques are used. Although an outer surface of the cap 307 and the driver 101 may not maintain sterility during handling and transport, an interior of the cavity 310, and any components disposed therein, i.e. portions that will make contact with a patient, will maintain sterility up until the moment of use.

In an embodiment, the system 100 can further include an extension set 160, or similar medical device(s), configured to be coupled to the needle hub 203 once the needle is placed to access a medullary cavity. In an embodiment, the system 100 can include an access kit (not shown) including additional equipment required to place and stabilize an access needle assembly 202. The access kit may include dressings, stabilization devices, sterilization wipes, gloves, combinations thereof, or the like. In an embodiment, the extension set 160 or access kit may be retained within a cavity defined by one of the driver body 101 or the cap 307. For example, as shown in FIG. 3, the handle of the driver 101 defines a cavity 170 configured to receive the extension set 160 disposed therein. The cavity 170 can be sealed with a lid 320 configured to allow sterilization of the extension set 160 disposed within the cavity 170, as described herein.

In an embodiment, the system 100 can be assembled with an access assembly 109 coupled to the driver 101, and have a cap 307 disposed thereover. The system 100 can further include an extension set 160 or access kit, disposed within the driver 101 and sealed therein by a lid 320. The system 100 can then be sterilized by EtO sterilization, Gamma sterilization, E-Beam sterilization, or the like, as described herein. Advantageously, this provides a system 100 that is ready to use and sterile, and maintains the portions that contact a patient in a sterile environment right up to the moment of use. Further, the system 100 provides all components required to access the medullary cavity as a single, a readily accessible, system 100, expediting the procedure.

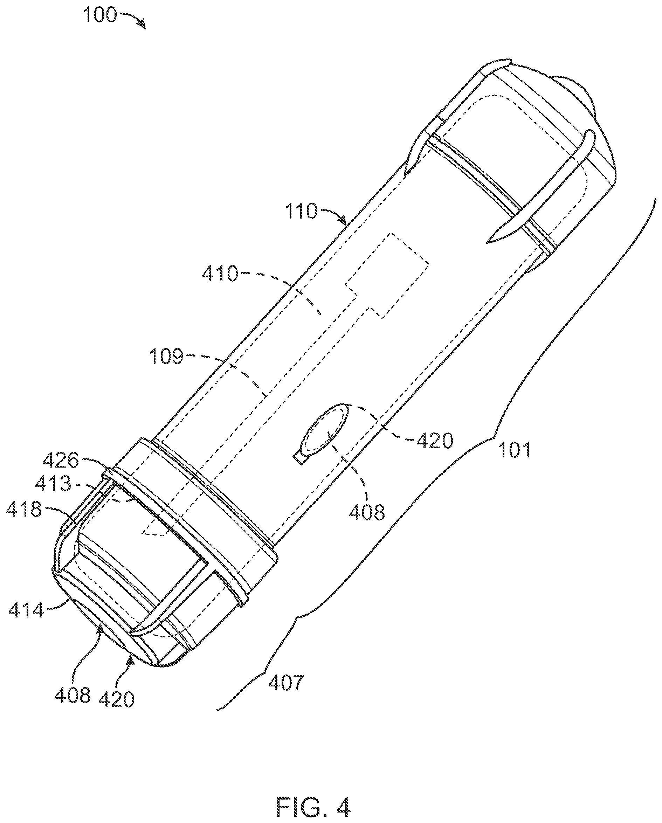

As shown in FIG. 4, in an embodiment, the intraosseous access system 100, includes a sterile packaging, such as cap 407, provided as a single-use system. The access assembly 109 is provided as already coupled with the driver 101, and is disposed in a retracted state where a portion of the access assembly 109 is disposed within the handle 110 of the driver 101. The handle 110 and cap 407 define a cavity 410 within which the access assembly 109 is disposed. The cap 407 and driver handle 110 maintains a sterile barrier between the surrounding environment and portions of the system 100 that make contact with the patient. Further, the cap 407 and driver handle 110 provide mechanical protection for the access assembly 109, disposed therein.

In an embodiment, the access assembly 109, or components thereof, are disposed in a retracted state within the driver 101, with the cap 407 covering an opening 413 disposed at a distal end of the driver 101. The access assembly 109 can pass through the opening 413 when transitioning from a retracted state to a deployed state. In an embodiment, the cap 407 is releasably attached to a distal end of the driver 101 by heat-sealing, bonding, adhesive, welding, or similar suitable techniques. In an embodiment, the cap 407 is coupled to the driver 101 by way of a threaded engagement between an inner surface of the cap 407 and an outer surface of the driver 101. In use, the user grasps outer surface of the cap 407 and can rotate the cap 407 relative to the driver 101 to twist and break the releasable securement, threaded portion, or combinations thereof, to release the cap 407 from the driver 101. In an embodiment, the cap 407 include fins 418 that provide mechanical strength to the cap 407 and provide a gripping feature. It will be appreciated that the outer surface of the cap 407 can further include additional textured surfaces, gripping features, and the like, as described herein.

In an embodiment, the cap 407 includes a tear away strip 426 that is defined by one or more tear lines, and includes a gripping feature, such as a tab or finger loop, as described herein. In use, a user pulls the gripping feature which causes the tear away strip 426 to separate from the intraosseous access system 100 and causes the cap 407 to separate from the driver 101.

In an embodiment, the cap 407 can be set aside and the access assembly 109 can be extended through the opening 413 to a deployed state and locked in place, ready for insertion into a patient. In an embodiment, the cap 407 can be coupled with the access assembly 109 and used as a grasping feature to pull the access assembly 109 through the opening 413 to a deployed state, while maintaining the sterility of the access assembly 109. In an embodiment, once the user has finished using the intraosseous access system 100 the needle 204 can then be retracted from the deployed state to a retracted state to contain the needle 204 for disposal. Optionally the cap 407 can be replaced over the opening 413 to fully contain the needle 204 for disposal. Advantageously, this can prevent accidental needle stick injuries and contains any blood disposed on the needle until the device can be safely disposed.

In an embodiment, the cap 407, driver 101, or combinations thereof, include an opening 408 communicating with the cavity 410. For example, as shown in FIG. 4, the opening 408 is disposed in a distal end 414 of the cap 407. The opening 408 includes a lid 420 attached thereto. In an embodiment, the lid 420 can be formed of a gas permeable material that allows EtO sterilization of the cavity 410, and any components disposed therein (e.g. access assembly 109) after the cap 407, access assembly 109, and driver 101 have been assembled. In an embodiment, lid 420, cap 407, driver 101, access assembly 109, combinations thereof, or the like, can be configured for Gamma or E-Beam sterilization either before or after assembly. Although an outer surface of the cap 407 and the driver 101 may not maintain sterility during handling and transport, an interior of the cavity 410, and associated components, will maintain sterility up until the moment of use.

Advantageously, the embodiment of intraosseous access system 100 shown in FIG. 4 defines a compact profile that is easy to store, transport and handle. This is of particular importance for emergency service personnel where capacity for carrying equipment is limited and rapid deployment is important. The system 100 maintains sterility of the patient contacting surfaces up until the moment and use and is quickly transitioned from a retracted, transportable state, to a deployable state, ready to be inserted into a patient.

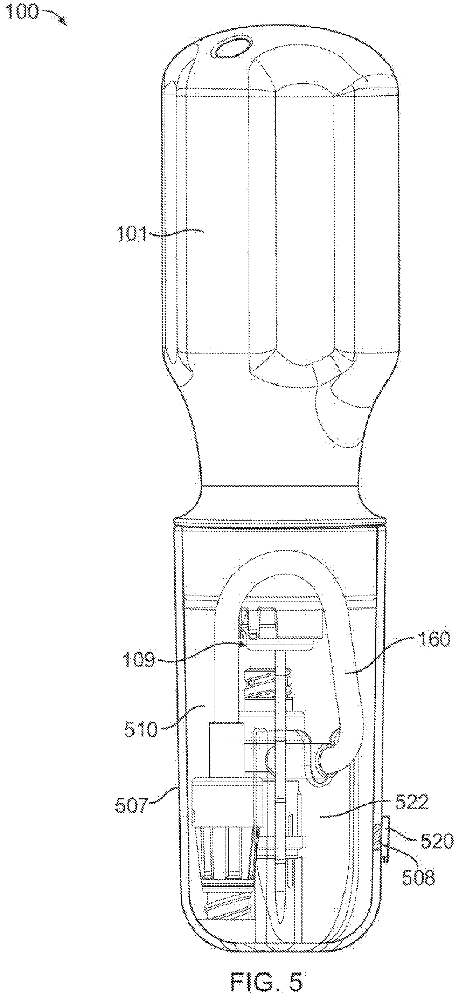

FIG. 5 shows an embodiment of an intraosseous access system 100 including a manual driver 101, an access assembly 109 coupled thereto, and a sterile packaging, e.g. cap 507 disposed thereover. In an embodiment, the cap 507 can define a cavity 510 configured to receive both the access assembly 109 and extension set 160 or access kit therein. In an embodiment, one of the body of the driver 101 or the cap 507 can include an opening 508 communicating with the cavity 510 of the cap 507. The opening 508 can be covered by a lid 520 configured to facilitate sterilization of both the access assembly 109 and extension set 160 disposed within the cavity 510 of the cap 507, after system 100 has been assembled, as described herein.

In an embodiment, the cap 507 can include a supporting structure 522 configured to releasably secure a portion of the extension set 160 and retain the extension set within the cap 507. Advantageously, the supporting structure 522 can prevent the extension set from falling out of the cap when the cap 507 is removed and the needle 204 is being placed. Instead, the extension set 160 is retained within the sterile environment within the cap 507 until it is removed by a user and attached to the needle hub 203.

Advantageously, the system 100 can provide both a fully assembled driver 101 and access assembly 109, as well as an extension set 160 or the like, within a sterile environment right up until the moment of use without requiring any additional packaging. The extension set 160, disposed within the cap 507 is readily available and does not require any additional steps to access, expediting the needle placement procedure.

The scope of the invention is defined by the claims.

## Claims

1. An intraosseous system including an intraosseous driver (101) configured to engage an access assembly (109), the intraosseous system comprising:
a sterile packaging (207) for containing the access assembly (109), the sterile packaging (207) comprising:
a cavity (210) configured to receive the access assembly (109);
a closed distal end (214);
a proximal opening (208) in communication with the cavity (210); and
a lid (220) disposed over the proximal opening (208) to seal the access assembly (109) in the cavity (210),
wherein the lid (220) includes a tear line (226) configured to allow a first portion of the lid (220) to separate from a second portion of the lid (220) and allow the access assembly (109) to pass through the proximal opening (208),
wherein the cavity (210) defines a first cross-sectional area configured to receive a needle portion of the access assembly (109), and a second cross-sectional area larger than the first cross-sectional area configured to receive a hub portion of the access assembly (109), and
wherein the second cross-sectional area includes an O-ring (230) configured to engage the hub portion of the access assembly (109) to provide a seal between a distal portion the cavity (210) and a proximal portion of the cavity (210).

2. The intraosseous system according to claim 1, wherein the lid (220) provides a sterile barrier to maintain the access assembly (109) in a sterile environment, and/or wherein the proximal opening (208) is configured to receive a distal portion of the driver (101) therethrough to engage the access assembly (109).

3. The intraosseous system according to claim 1 or 2, wherein the second cross-sectional area releasably engages the hub portion of the access assembly (109) in an interference engagement, protrusion and detent engagement, threaded engagement, or adhesive engagement.

4. The intraosseous system according to any claim of claims 1-3, wherein the lid (220) is formed of a gas-permeable, organic material, synthetic material, woven material, non-woven material, polymer, co-polymer, olefin fiber, polyethylene, high density polyethylene ("HDPE"), and/or wherein the lid (220) provides a physical and sterile barrier as well as allowing for EtO sterilization of the access assembly (109) disposed within the cavity (210).

5. The intraosseous system according to any claim of claims 1-4, wherein the lid (220) is releasably coupled to the proximal opening (208) with one of heat-sealing, bonding, adhesive, or welding, and/or wherein the lid (220) includes one of a finger loop or a pull tab configured to facilitate separation of the lid (220) from the sterile packaging (207).

6. The intraosseous system according to any claim of claims 1-5, wherein the sterile packaging (207) includes a flange (216) extending radially from a proximal edge of the cap.

7. The intraosseous system according to any claim of claims 1-6, wherein the sterile packaging (207) further includes a fin (218) extending longitudinally along an outer surface thereof and configured to provide structural support.

8. A method of preparing an intraosseous access device for use, comprising:
providing an access assembly (109) disposed within a cavity (210) of a sterile packaging (207), and including a lid (220) disposed over a proximal opening (208) that communicates with the cavity (210), to provide a sterile environment therein;
urging a coupling interface of an intraosseous driver (101) through the proximal opening (208) to engage a coupling interface of the access assembly (109);
withdrawing the sterile packaging (207) distally to expose the access assembly (109); and
whereby urging the coupling interface of the driver (101) through a tear line (226) disposed in the lid (220), the tear line (226) configured to separate a first portion of the lid (220) from a second portion of the lid (220), allows the coupling interface of the driver (101) to pass therebetween,
wherein the cavity (210) includes an O-ring (230) disposed on an inner surface thereof and configured to engage the hub portion to provide a seal between a distal portion of the sterile packaging (207) and a proximal portion of the sterile packaging (207).

9. The method according to claim 8, further including at least one of the following steps:
disengaging a hub portion of the access assembly (109) from an interior surface of the sterile packaging (207), the hub portion engaging the sterile packaging (207) in an interference engagement, protrusion and detent engagement, threaded engagement, or adhesive engagement,
removing the lid (220) from the sterile packaging (207) prior to urging the coupling interface of the driver through the proximal opening (208), and
pulling one of a finger loop (222) or a pull tab (224) coupled to the lid (220) to separate the lid (220) from the sterile packaging (207).

10. The method according to either of claims 8 or 9, wherein the lid (220) is formed of a gas-permeable material, organic material, synthetic material, woven material, non-woven material, polymer, co-polymer, olefin fiber, polyethylene, high density polyethylene ("HDPE").

11. The method according to any claim of claims 8-10, wherein the lid (220) provides a physical and sterile barrier as well as allowing for EtO sterilization of the sterile packaging (207), and the access assembly (109) disposed therein, and/or wherein the lid (220) is coupled to the sterile packaging (207) with one of heat-sealing, bonding, adhesive, or welding.

12. The method according to any claim of claims 8-11, wherein the sterile packaging (207) includes a flange (216) extending radially from a proximal edge of the sterile packaging (207).

## Patentansprüche

1. Intraossäres System, das einen intraossären Treiber (101) einschließt, der ausgebildet ist, um in eine Zugangsbaugruppe (109) einzugreifen, wobei das intraossäre System Folgendes umfasst:
eine sterile Verpackung (207) zum Enthalten der Zugangsbaugruppe (109), wobei die sterile Verpackung (207) Folgendes umfasst:
einen Hohlraum (210), der ausgebildet ist, um die Zugangsbaugruppe (109) zu empfangen;
ein geschlossenes distales Ende (214);
eine proximale Öffnung (208) in Kommunikation mit dem Hohlraum (210); und
einen Deckel (220), der über der proximalen Öffnung (208) angeordnet ist, um die Zugangsbaugruppe (109) im Hohlraum (210) abzudichten,
wobei der Deckel (220) eine Aufreißlinie (226) einschließt, die ausgebildet ist, um zu erlauben, einen ersten Abschnitt des Deckels (220) von einem zweiten Abschnitt des Deckels (220) zu trennen, und zu erlauben, die Zugangsbaugruppe (109) durch die proximale Öffnung (208) zu passieren,
wobei der Hohlraum (210) eine erste Querschnittsfläche, die ausgebildet ist, um einen Nadelabschnitt der Zugangsbaugruppe (109) zu empfangen, und eine zweite Querschnittsfläche definiert, die größer ist als die erste Querschnittsfläche, die ausgebildet ist, um einen Nabenabschnitt der Zugangsbaugruppe (109) zu empfangen, und
wobei die zweite Querschnittsfläche einen O-Ring (230) einschließt, der ausgebildet ist, um mit dem Nabenabschnitt der Zugangsbaugruppe (109) in Eingriff zu kommen, um eine Abdichtung zwischen einem distalen Abschnitt des Hohlraums (210) und einem proximalen Abschnitt des Hohlraums (210) bereitzustellen.

2. Intraossäres System nach Anspruch 1, wobei der Deckel (220) eine sterile Barriere bereitstellt, um die Zugangsbaugruppe (109) in einer sterilen Umgebung zu halten, und/oder wobei die proximale Öffnung (208) ausgebildet ist, um einen distalen Abschnitt des Treibers (101) durch sich hindurch zu empfangen, um mit der Zugangsbaugruppe (109) in Eingriff zu kommen.

3. Intraossäres System nach Anspruch 1 oder 2, wobei die zweite Querschnittsfläche mit dem Nabenabschnitt der Zugangsbaugruppe (109) in einem Interferenzeingriff, einem Vorsprung- und Rasteingriff, einem Gewindeeingriff oder einem Adhäsionseingriff lösbar in Eingriff steht.

4. Intraossäres System nach einem der Ansprüche 1-3, wobei der Deckel (220) aus einem gasdurchlässigen organischen Material, einem synthetischen Material, einem gewobenen Material, einem vliesartigen Material, einem Polymer, einem Copolymer, einer Olefinfaser, Polyethylen, Polyethylen hoher Dichte ("HDPE") gebildet ist und/oder wobei der Deckel (220) eine physikalische und sterile Barriere bereitstellt sowie eine EtO-Sterilisation der innerhalb des Hohlraums (210) angeordneten Zugangsbaugruppe (109) erlaubt.

5. Intraossäres System nach einem Anspruch der Ansprüche 1-4, wobei der Deckel (220) mit einem aus Heißsiegeln, Bonding, Kleben oder Schweißen mit der proximalen Öffnung (208) lösbar gekoppelt ist und/oder wobei der Deckel (220) eine Fingerschlaufe oder eine Zuglasche einschließt, die ausgebildet sind, um die Trennung des Deckels (220) von der sterilen Verpackung (207) zu erleichtern.

6. Intraossäres System nach einem Anspruch der Ansprüche 1-5, wobei die sterile Verpackung (207) einen Flansch (216) einschließt, der sich radial von einem proximalen Rand der Kappe erstreckt.

7. Intraossäres System nach einem Anspruch der Ansprüche 1-6, wobei die sterile Verpackung (207) weiter eine Lamelle (218) einschließt, die sich longitudinal entlang einer Außenoberfläche derselben erstreckt und ausgebildet ist, um strukturelle Unterstützung bereitzustellen.

8. Verfahren zum Vorbereiten einer intraossären Zugangsvorrichtung zur Verwendung, umfassend:
Bereitstellen einer Zugangsbaugruppe (109), die innerhalb eines Hohlraums (210) einer sterilen Verpackung (207) angeordnet ist, und einen Deckel (220) einschließt, der über einer proximalen Öffnung (208) angeordnet ist, die mit dem Hohlraum (210) kommuniziert, um darin eine sterile Umgebung bereitzustellen;
Drängen einer Kopplungsschnittstelle eines intraossären Treibers (101) durch die proximale Öffnung (208), um mit einer Kopplungsschnittstelle der Zugangsbaugruppe (109) in Eingriff zu kommen;
Zurückziehen der sterilen Verpackung (207) in distaler Richtung, um die Zugangsbaugruppe (109) freizulegen; und
wobei Drängen der Kopplungsschnittstelle des Treibers (101) durch eine im Deckel (220) angeordnete Aufreißlinie (226), wobei die Aufreißlinie (226) ausgebildet ist, um einen ersten Abschnitt des Deckels (220) von einem zweiten Abschnitt des Deckels (220) zu trennen, der Kopplungsschnittstelle des Treibers (101) erlaubt, dazwischen zu passieren,
wobei der Hohlraum (210) einen O-Ring (230) einschließt, der an einer Innenoberfläche desselben angeordnet und ausgebildet ist, um mit dem Nabenabschnitt in Eingriff zu kommen, um eine Abdichtung zwischen einem distalen Abschnitt der sterilen Verpackung (207) und einem proximalen Abschnitt der sterilen Verpackung (207) bereitzustellen.

9. Verfahren nach Anspruch 8, weiter einschließend mindestens einen der folgenden Schritte:
außer Eingriff bringen eines Nabenabschnitts der Zugangsbaugruppe (109) von einer Innenoberfläche der sterilen Verpackung (207), wobei der Nabenabschnitt mit der sterilen Verpackung (207) in einem Interferenzeingriff, einem Vorsprung- und Rasteingriff, einem Gewindeeingriff oder einem Klebeeingriff in Eingriff steht,
Entfernen des Deckels (220) von der sterilen Verpackung (207) vor dem Drängen der Kopplungsschnittstelle des Treibers durch die proximale Öffnung (208), und
Ziehen an einem aus einer Fingerschlaufe (222) oder einer Zuglasche (224), die mit dem Deckel (220) gekoppelt sind, um den Deckel (220) von der sterilen Verpackung (207) zu trennen.

10. Verfahren nach einem der Ansprüche 8 oder 9, wobei der Deckel (220) aus einem gasdurchlässigen Material, einem organischen Material, einem synthetischen Material, einem gewebten Material, einem vliesartigen Material, einem Polymer, einem Copolymer, einer Olefinfaser, Polyethylen oder Polyethylen hoher Dichte ("HDPE") gebildet ist.

11. Verfahren nach einem Anspruch der Ansprüche 8-10, wobei der Deckel (220) eine physikalische und sterile Barriere bereitstellt sowie eine EtO-Sterilisation der sterilen Verpackung (207) und der darin angeordneten Zugangsbaugruppe (109) erlaubt, und/oder wobei der Deckel (220) mit einem aus Heißsiegeln, Bonding, Kleben oder Schweißen mit der sterilen Verpackung (207) gekoppelt ist.

12. Verfahren nach einem Anspruch der Ansprüche 8-11, wobei die sterile Verpackung (207) einen Flansch (216) einschließt, der sich radial von einem proximalen Rand der sterilen Verpackung (207) erstreckt.

## Revendications

1. Système intraosseux incluant un dispositif d'entraînement intraosseux (101) configuré pour venir en prise avec un ensemble d'accès (109), le système intraosseux comprenant :
un emballage stérile (207) pour contenir l'ensemble d'accès (109), l'emballage stérile (207) comprenant :
une cavité (210) configurée pour recevoir l'ensemble d'accès (109) ;
une extrémité distale fermée (214) ;
une ouverture proximale (208) en communication avec la cavité (210) ; et
un opercule (220) disposé sur l'ouverture proximale (208) pour assurer l'étanchéité de l'ensemble d'accès (109) dans la cavité (210),
dans lequel l'opercule (220) inclut une ligne de déchirure (226) configurée pour permettre à une première partie de l'opercule (220) de se séparer d'une deuxième partie de l'opercule (220) et permettre à l'ensemble d'accès (109) de passer à travers l'ouverture proximale (208),
dans lequel la cavité (210) définit une première aire de section transversale configurée pour recevoir une partie d'aiguille de l'ensemble d'accès (109), et une deuxième aire de section transversale plus grande que la première aire de section transversale configurée pour recevoir une partie de moyeu de l'ensemble d'accès (109), et
dans lequel la deuxième aire de section transversale inclut un joint torique (230) configuré pour venir en prise avec la partie de moyeu de l'ensemble d'accès (109) afin d'assurer une étanchéité entre une partie distale de la cavité (210) et une partie proximale de la cavité (210).

2. Système intraosseux selon la revendication 1, dans lequel l'opercule (220) fournit une barrière stérile pour maintenir l'ensemble d'accès (109) dans un environnement stérile, et/ou dans lequel l'ouverture proximale (208) est configurée pour recevoir une partie distale du dispositif d'entraînement (101) à travers celle-ci pour venir en prise avec l'ensemble d'accès (109).

3. Système intraosseux selon la revendication 1 ou la revendication 2, dans lequel la deuxième aire de section transversale vient en prise avec la partie de moyeu de l'ensemble d'accès (109) selon une mise en prise par interférence, une mise en prise en saillie et en encliquetage, une mise en prise filetée, ou une mise en prise adhésive.

4. Système intraosseux selon l'une quelconque des revendications 1 à 3, dans lequel l'opercule (220) est formé d'un matériau perméable aux gaz, d'un matériau organique, d'un matériau synthétique, d'un matériau tissé, d'un matériau non tissé, d'un polymère, d'un copolymère, de fibres d'oléfine, d'un polyéthylène, d'un polyéthylène haute densité (« HDPE »), et/ou dans lequel
l'opercule (220) fournit une barrière physique et stérile tout en permettant la stérilisation à l'EtO de l'ensemble d'accès (109) disposé à l'intérieur de la cavité (210).

5. Système intraosseux selon l'une quelconque des revendications 1 à 4, dans lequel l'opercule (220) est couplé de manière amovible à l'ouverture proximale (208) avec l'un de thermoscellage, de collage, de collage par adhésif, ou de soudage, et/ou dans lequel l'opercule (220) inclut l'un ou l'une d'un anneau ou d'une tirette configuré(e) pour faciliter la séparation de l'opercule (220) de l'emballage stérile (207).

6. Système intraosseux selon l'une quelconque des revendications 1 à 5, dans lequel l'emballage stérile (207) inclut une collerette (216) s'étendant radialement à partir d'un bord proximal du capuchon.

7. Système intraosseux selon l'une quelconque des revendications 1 à 6, dans lequel l'emballage stérile (207) inclut en outre une nervure (218) s'étendant longitudinalement le long d'une surface externe de celui-ci et configurée pour fournir un support structurel.

8. Procédé de préparation d'un dispositif d'accès intraosseux destiné à l'utilisation, comprenant :
la fourniture d'un ensemble d'accès (109) disposé à l'intérieur d'une cavité (210) d'un emballage stérile (207), et incluant un opercule (220) disposé sur une ouverture proximale (208) qui communique avec la cavité (210), pour fournir un environnement stérile dans celui-ci ;
la poussée d'une interface de couplage d'un dispositif d'entraînement intraosseux (101) à travers l'ouverture proximale (208) pour venir en prise avec une interface de couplage de l'ensemble d'accès (109) ;
le retrait de l'emballage stérile (207) distalement pour exposer l'ensemble d'accès (109) ; et
par lequel la poussée de l'interface de couplage du dispositif d'entraînement (101) à travers une ligne de déchirure (226) disposée dans l'opercule (220), la ligne de déchirure (226) étant configurée pour séparer une première partie de l'opercule (220) d'une deuxième partie de l'opercule (220), permet à l'interface de couplage du dispositif d'entraînement (101) de passer entre celles-ci,
dans lequel la cavité (210) inclut un joint torique (230) disposé sur une surface interne de celle-ci et configuré pour venir en prise avec la partie de moyeu afin d'assurer une étanchéité entre une partie distale de l'emballage stérile (207) et une partie proximale de l'emballage stérile (207).

9. Procédé selon la revendication 8, incluant en outre au moins l'une des étapes suivantes :
la mise hors prise d'une partie de moyeu de l'ensemble d'accès (109) depuis une surface interne de l'emballage stérile (207), la partie de moyeu venant en prise avec l'emballage stérile (207) selon une mise en prise par interférence, une mise en prise en saillie et en encliquetage, une mise en prise filetée, ou une mise en prise adhésive,
le retrait de l'opercule (220) de l'emballage stérile (207) avant de pousser l'interface de couplage du dispositif d'entraînement à travers l'ouverture proximale (208), et
la traction de l'un ou de l'une d'un anneau (222) ou d'une tirette (224) couplé(e) à l'opercule (220) pour séparer l'opercule (220) de l'emballage stérile (207).

10. Procédé selon l'une ou l'autre des revendications 8 ou 9, dans lequel l'opercule (220) est formé d'un matériau perméable aux gaz, d'un matériau organique, d'un matériau synthétique, d'un matériau tissé, d'un matériau non tissé, d'un polymère, d'un copolymère, de fibres d'oléfine, d'un polyéthylène, d'un polyéthylène haute densité (« HDPE »).

11. Procédé selon l'une quelconque des revendications 8 à 10, dans lequel l'opercule (220) fournit une barrière physique et une barrière stérile tout en permettant la stérilisation à l'EtO de l'emballage stérile (207), et de l'ensemble d'accès (109) disposé dans celui-ci, et/ou dans lequel l'opercule (220) est couplé à l'emballage stérile (207) avec l'un de thermoscellage, de collage, de collage par adhésif, ou de soudage.

12. Procédé selon l'une quelconque des revendications 8 à 11, dans lequel l'emballage stérile (207) inclut une collerette (216) s'étendant radialement à partir d'un bord proximal de l'emballage stérile (207).
